# EUROPEAN PATENT APPLICATION

(11) **EP 4 778 897 A1**
(43) Date of publication of application: **22.07.2026**
(21) Application number: 24865438.6
(22) Date of filing: 10.09.2024
(51) Int. Cl.: C03C 12/00, A61K 8/25, A61K 8/26, A61Q 1/12

(54) **GLASS POWDER**

(30) Priority: 15.09.2023 JP 2023149887
(71) Applicant: Nippon Sheet Glass Company, Limited, Tokyo 108-6321 (JP)
(72) Inventor: HORIGUCHI, Haruko, Tokyo 108-6321 (JP); MIKAMI, Shinji, Tokyo 108-6321 (JP)
(74) Representative: Cabinet Beau de Loménie
(86) International application number: PCT/JP2024/032362
(87) International publication number: WO 2025/057938

(57) **Abstract**

A glass powder in which D50 is 1 µm or more and 15 µm or less and D50/2 is 8% or more is provided. Additionally, a glass powder having a hardness A of 1.5 or more as measured for the glass powder molded into a press-molded body having a height of 5 mm is provided. D50/2 is a proportion of a cumulative volume from a small-particle side up to a particle size equal to 1/2 of D50 in a cumulative volume particle size distribution measured by a laser diffraction-scattering method. The press-molded body is obtained by applying a 4 MPa pressure, and the hardness A is measured using a type A durometer specified in JIS K 6253-2012.

## Description

### TECHNICAL FIELD

The present invention relates to a glass powder. More specifically, the present invention relates to a glass powder suitable for, for example, cosmetics.

### BACKGROUND ART

Sometimes, powders are included in cosmetics to, for example, enhance the slipperiness and impart a light scattering effect. Powders are frequently used for makeup cosmetics typified by foundations. As organic powders, silicone, cellulose, nylon, and the like are used. As inorganic powders, silica, glass, and the like are used. Patent Literature 1 discloses, as a cosmetic, a transparent solid composition including a powder including spherical particles having an average particle diameter of 3 to 30 µm.

### CITATION LIST

### Patent Literature

Patent Literature 1: JP 2005-213145 A

### SUMMARY OF INVENTION

### Technical Problem

Unlike organic powders, inorganic powders, such as glass powders, do not produce microplastics after discarded. However, inorganic powders may give dryness or roughness to skin. Inorganic powders may also impair the moldability of pressed powder-type cosmetics. Hence, the present invention aims to provide a glass powder suitable for cosmetics.

### Solution to Problem

In a first aspect, the present invention provides a glass powder, wherein D50 is 1 µm or more and 15 µm or less and D50/2 is 8% or more, where D50 is a particle diameter at which a cumulative volume from a small-particle side is 50% in a particle size distribution measured by a laser diffraction-scattering method, and D50/2 is a proportion of a cumulative volume from the small-particle side up to a particle size equal to 1/2 of the D50 in the particle size distribution.

In a second aspect, the present invention provides a glass powder having a hardness A of 1.5 or more as measured for the glass powder molded into a cylindrical press-molded body having a height of 5 mm, the press-molded body being obtained by filling a mold having a cavity in a shape of a cylinder with the glass powder and applying a 4 MPa pressure to the filled glass powder in a height direction of the cylinder, the hardness A being measured using a type A durometer specified in Japanese Industrial Standards (JIS) K 6253-2012.

### Advantageous Effects of Invention

The present invention provides a glass powder suitable for cosmetics. The present invention can exhibit at least one effect selected from i) and ii) at least in a preferable embodiment:
i) easily providing a soft and silky feel to skin; and
ii) minimally impairing the moldability of pressed powder-type cosmetics.

### BRIEF DESCRIPTION OF DRAWING

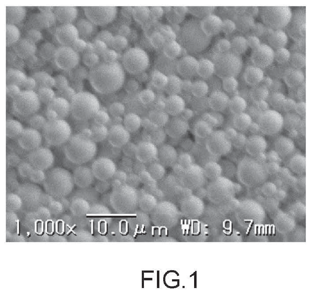

FIG. 1 showing the result of scanning electron microscopic (SEM) observation of a glass powder according to an example of the present invention.

### DESCRIPTION OF EMBODIMENTS

Hereinafter, the present invention will be described in detail. The following description is not intended to limit the present invention to specific embodiments. Herein, the term "main component" refers to a component with the highest content on a mass basis. The term "spherical shape" refers to a shape of a particle having a ratio R2/R1 of 0.8 or more, the ratio R2/R1 being a ratio of a diameter R2 orthogonal to a longest diameter R1 to the diameter R1. The term "perfect spherical shape" refers to a shape of a particle having a ratio R2/R1 of 0.9 or more, the ratio R2/R1 being the ratio of the diameter R2 orthogonal to the longest diameter R1 to the diameter R1. Whether a shape corresponds to the "spherical shape" or the "perfect spherical shape" can be determined using an image obtained by SEM observation. A glass powder "consisting substantially of" a particular glass particle means that 90% or more, or even 95% or more of glass particles forming the glass powder correspond to the particular glass particle on a number basis. Note that the proportion on a number basis can be determined from arbitrarily chosen 50 particles. The upper and lower limits of numerical values given below are not described individually, and can be employed in any combination.

### (Particle Size Distribution)

D50 of the glass powder of the present embodiment may be 1 µm or more, 2 µm or more, 3 µm or more, or even 4 µm or more. An excessively small D50 may reduce the dispersibility of the glass powder in cosmetics. D50 may be 15 µm or less, 13 µm or less, 11 µm or less, or even 10.5 µm or less. An excessively large D50 may prevent maintaining a pleasant feel on skin. D50/2 may be 8% or more, 10% or more, 12% or more, 14% or more, even 16% or more, or, in some cases, 18% or more. D50/2 is a measure of a proportion of particles having relatively small particle diameters. The upper limit of D50/2 is not limited to a particular value, and may be 40% or less, 35% or less, or even 30% or less.

The glass powder having not excessively small a D50/2 easily provides a soft and silky feel to skin. The reason is thought to be that the glass powder contacts skin, with small particles sufficiently interposed between relatively large particles. Glass powders, which are harder than organic powders, are inherently less advantageous in terms of providing a soft and silky feel. Controlling a particle size distribution of the glass powder can allow the glass powder to provide a soft and silky feel, and that is advantageous in mass production in that the need for surface modification of inorganic powders can be eliminated.

The glass powder having not excessively small a D50/2 is also superior in that the glass powder minimally impairs the moldability of cosmetics. The moldability of the glass powder itself can be used as an evaluation of the degree of an impact of the glass powder on the moldability of cosmetics. Hardnesses of a press-molded body of the glass powder can be used as an evaluation of the moldability of the glass powder.

The lower limit of D10 of the glass powder is not particularly limited, and may be 0.3 µm or more, 0.7 µm or more, or even 1 µm or more. The upper limit of D10 is not particularly limited, either, and may be 8 µm or less, 5 µm or less, or even 3 µm or less. The lower limit of D90 of the glass powder is not particularly limited, and may be 1 µm or more, 5 µm or more, or even 7 µm or more. The upper limit of D90 is not particularly limited, either, and may be 50 µm or less, 40 µm or less, or even 25 µm or less. It should be noted that D10 and D90 can be determined as values obtained by interpreting "50%" for D50 as "10%" and "90%", respectively.

A ratio D90/D10 of D90 to D10 is a measure of the width of the particle size distribution. D90/D10 is not limited to a particular value, and may be 4 or more, 5 or more, or even 6 or more. D90/D10 is not limited to a particular value, and may be 50 or less, 30 or less, 15 or less, 12 or less, even 10 or less, or, in some cases, 9 or less. D90/D10 in an appropriate range, particularly 4 or more and 20 or less, 5 or more and 10 or less, or even 5 or more and 9 or less, is appropriate to reduce the degree of the impact on the moldability of cosmetics.

### (Hardnesses of Press-Molded Body)

A hardness A of a press-molded body of the glass powder of the present embodiment may be 1.5 or more, 2 or more, even 2.2 or more, in some cases, 2.5 or more, or particularly 3 or more. A high hardness of the press-molded body of the glass powder indicates a reduction of the degree of the impact on the moldability of cosmetics including the glass powder. The upper limit of the hardness A of the press-molded body of the glass powder is, for example, but not particularly limited to, 20 or less, or even 10 or less. It should be noted that the hardness A is based on a hardness measured using a type A durometer. When described as a hardness F measured using an ASKER durometer type F, a hardness (hardness F) of the press-molded body of the glass powder of the present embodiment may be 60 or more, 70 or more, 80 or more, 90 or more, or even 95 or more. Details of the method for producing the press-molded body being a test sample and the method for evaluating the hardnesses A and F are described in EXAMPLES. As for a glass powder that can form a press-molded body having a high hardness, it is believed that relatively small particles are sufficiently interposed between relatively large particles.

### (Composition of Glass Powder)

A composition of the glass powder is not limited to a particular one, and may be a composition including an oxide, such as silicon oxide, as its main component. A glass composition including silicon oxide as its main component may be a composition called, for example, soda-lime glass, borosilicate glass, or aluminosilicate glass. The glass composition may be a composition corresponding to at least one selected from the group consisting of aluminum calcium copper sodium borosilicate, calcium aluminum borosilicate, calcium sodium borosilicate, and calcium titanium borosilicate as expressed by International Nomenclature of Cosmetic Ingredients (INCI) names. The glass composition may be a composition corresponding to at least one selected from the group consisting of calcium aluminum borosilicate, calcium sodium borosilicate, and calcium titanium borosilicate as expressed by INCI names. The glass composition may be a composition corresponding to at least one selected from the group consisting of calcium aluminum borosilicate and calcium sodium borosilicate as expressed by INCI names. In each of the above compositions including "borosilicate" in their INCI names, the boron oxide content may be 0 to 13% on a mass basis. That is, even a boron oxide-free glass composition can correspond to a composition including "borosilicate" in its INCI name. The composition of the glass powder may be one in which the calcium oxide content is higher than that in an ordinary soda-lime glass, specifically, one having a calcium oxide content of 16% or more, or even 18% or more on a mass basis. The composition of the glass powder may be one in which the aluminum oxide content is higher than that in an ordinary soda-lime glass, specifically, one having an aluminum oxide content of 5% or more, or even 8% or more on a mass basis. The composition of the glass powder may be one in which the sodium oxide content is lower than that in an ordinary soda-lime glass, specifically, one having an aluminum oxide content of 10% or less, or even 7% or less on a mass basis. In addition, a glass composition having a softening point higher than that of soda-lime glass, specifically, a glass composition having a softening point of 780°C or higher, 800°C or higher, or even 830°C or higher, is suitable as the glass powder of the present embodiment. A softening point is a temperature at which a glass has a viscosity of 10^{7.6} dPa·s.

### (Shape of Particle Forming Glass Powder, Etc.)

A shape of the particle forming the glass powder is not limited to a particular shape, and may be a spherical shape or a perfect spherical shape. The glass powder may consist substantially of a glass particle having a spherical shape. The glass powder may consist substantially of a glass particle having a perfect spherical shape. However, the glass powder of the present embodiment can have any of a variety of shapes. The glass powder may consist substantially of a solid glass particle. The solid glass particle is more suitable than a hollow glass particle in terms of providing a sufficient soft and silky feel.

### (Glass Powder Manufacturing Method)

In the present embodiment, the glass powder can be obtained, for example, by a manufacturing method including pulverizing a raw material glass powder. The pulverization step is not particularly limited, and can be performed, for example, using a known mill. The raw material glass powder is not limited to a particular one, either, and a raw material powder having any of a variety of shapes, such as a pellet shape, a flaky shape, and a spherical shape, can be employed.

Pulverization conditions for adjusting the particle size depend on the pulverization process. For example, as for ball milling, it is well known that pulverization conditions include a pulverization time, a rotating speed, a ball filling rate, a ball diameter, and the like. After the pulverization, a glass powder having a diameter larger or smaller than a given diameter may be removed using a sieve, a filter, or the like. The adjustment itself of the particle size distribution can be performed by applying a known technique.

The glass powder manufacturing method may further include spheroidizing the glass powder obtained by the pulverization. The spheroidization step can be performed, for example, by heating the glass powder. The heating temperature can be determined by taking into account, for example, the composition of the glass powder and the particle size distribution thereof. At too high a heating temperature, the glass powder having a small particle diameter can foam. A moderately low heating temperature is suitable for maintaining a proportion of relatively small particles forming the glass powder. Studies by the present inventors have revealed that heating temperatures traditionally employed and suitable for enhancing the production efficiency decrease the proportion of relatively small particles, which can be represented by D50/2.

### (Cosmetics)

Cosmetics in which the glass powder of the present embodiment is to be included are, for example, but not particularly limited to, makeup cosmetics, such as foundations and face powders. The shapes of such cosmetics are not limited to particular shapes, either. The glass powder of the present embodiment is suitable for being included in solid cosmetics.

The content of the glass powder of the present embodiment in a cosmetic may be, for example, 0.1% or more, 5% or more, or even 10% or more on a mass basis. The upper limit of this content is not limited to a particular value, and may be 90% or less, or even 50% or less.

As described above, the present embodiment provides the following techniques.

A first technique is a glass powder, wherein D50 is 1 µm or more and 15 µm or less and D50/2 is 8% or more, where D50 is a particle diameter at which a cumulative volume from a small-particle side is 50% in a particle size distribution measured by a laser diffraction-scattering method, and D50/2 is a proportion of a cumulative volume from the small-particle side up to a particle size equal to 1/2 of the D50 in the particle size distribution.

A second technique is the glass powder according to the first technique having a hardness A of 1.5 or more as measured for the glass powder molded into a cylindrical press-molded body having a height of 5 mm, the press-molded body being obtained by filling a mold having a cavity in a shape of a cylinder with the glass powder and applying a 4 MPa pressure to the filled glass powder in a height direction of the cylinder, the hardness A being measured using a type A durometer specified in Japanese Industrial Standards (JIS) K 6253-2012.

A third technique is a glass powder having a hardness A of 1.5 or more as measured for the glass powder molded into a cylindrical press-molded body having a height of 5 mm, the press-molded body being obtained by filling a mold having a cavity in a shape of a cylinder with the glass powder and applying a 4 MPa pressure to the filled glass powder in a height direction of the cylinder, the hardness A being measured using a type A durometer specified in Japanese Industrial Standards (JIS) K 6253-2012.

A fourth technique is the glass powder according to any one of the first to third techniques consisting substantially of a glass particle having a spherical shape, where the spherical shape is a shape having a ratio R2/R1 of 0.8 or more, the ratio R2/R1 being a ratio of a diameter R2 orthogonal to a longest diameter R1 to the diameter R1 in an observation image of the glass particle observed using a scanning electron microscope.

A fifth technique is the glass powder according to any one of the first to fourth technique, wherein a ratio D90/D10 of D90 to D10 is 4.0 or more, where D10 is a particle diameter at which a cumulative volume from a small-particle side is 10% in a particle size distribution measured by a laser diffraction-scattering method, and D90 is a particle diameter at which the cumulative volume from the small-particle side is 90% in the particle size distribution.

A sixth technique is the glass powder according to any one of the first to fifth techniques having a glass composition having a softening point of 780°C or higher.

A seventh technique is the glass powder according to the first technique having a hardness F of 60 or more as measured for the glass powder molded into a cylindrical press-molded body having a height of 5 mm, the press-molded body being obtained by filling a mold having a cavity in a shape of a cylinder with the glass powder and applying a 4 MPa pressure to the filled glass powder in a height direction of the cylinder, the hardness F being measured using an ASKER durometer type F.

An eighth technique is a cosmetic including the glass powder according to any one of the first to seventh techniques.

The present embodiment also provides a ninth techniques given below.

The ninth techniques is a glass powder having a hardness F of 60 or more as measured for the glass powder molded into a cylindrical press-molded body having a height of 5 mm, the press-molded body being obtained by filling a mold having a cavity in a shape of a cylinder with the glass powder and applying a 4 MPa pressure to the filled glass powder in a height direction of the cylinder, the hardness F being measured using an ASKER durometer type F.

### EXAMPLES

Hereinafter, the present invention will be described in more detail with reference to examples. First, specific evaluation methods will be described.

### (Particle Size Distribution)

A particle size distribution of a glass powder was measured by a laser diffraction-scattering method. A particle diameter distribution measurement device (MT3300EX II manufactured by Microtrac MRB) was used for the measurement. Particle diameters at which cumulative volumes from the small-particle side are 10%, 50%, and 90% were measured as D10, D50, and D90, respectively. Moreover, the proportion of the cumulative volume of particles from the small-particle side up to a particle size equal to one-half of D50 was obtained as D50/2. Furthermore, D90/D10 was calculated.

### (Sensory Evaluation)

A small amount of a glass powder was taken on a finger and applied to the back of a hand to evaluate the feel of the powder and the skin dryness. The feel was evaluated as either soft or smooth. The dryness was evaluated on a three-level scale: G, not dry; F, slightly dry; and NG, dry.

### (Shape Maintenance and Hardness of Press-Molded Body)

### •Molding of press-molded body

A glass powder was compression-molded into a cylindrical press-molded body using a mold. The press-molded body had a cylindrical shape having a 58 mm-diameter circular bottom and having a height of 5 mm. The compression molding was performed by applying a 4 MPa pressure in a height direction of the cylinder.

### •Shape maintenance

A 10 kPa pressure was applied to a circular area within 10 mm from the center of the bottom face of the press-molded body, and a change of the shape of the press molding body was observed. The result was evaluated on a two-level scale: G, resistant to deformation; and NG, easy to deform.

### •Hardness

The hardnesses A and F of the bottom face of the press-molded body were measured using a type A durometer specified in JIS K 6253-2012 and an ASKER durometer type F manufactured by KOBUNSHI KEIKI CO., LTD. The measurement time was one second. In other words, indicated values obtained within one second after the durometers came into contact with the press-molded body were employed as the hardnesses.

### (Example 1)

Glass particles having a glass composition corresponding to calcium aluminum borosilicate were pulverized with a ball mill to give a glass powder. Table 1 shows the glass composition of the calcium aluminum borosilicate. Note that this glass composition also corresponds to calcium sodium borosilicate and calcium titanium borosilicate, and thus can be expressed as any one of the three including calcium aluminum borosilicate. Additionally, this glass composition has a softening point of 876°C. Pulverization conditions for the ball mill were adjusted so that D50 of the glass powder would be 6 µm. The pulverized glass powder had a shape not corresponding to the spherical shape. The resulting glass powder was then spheroidized using a flame spheroidizing system ("CERAMELT" by Taiyo Nippon Sanso Corporation). The burner combustion temperature of the spheroidizing system was adjusted within the range of 2000 to 2400°C. The glass powder was fed at 10 kg/h using a raw material feeder. The fed glass powder was heated to be spheroidized. The spheroidized glass particles were collected using a cyclone. On the other hand, a fine powder having a particle diameter of 100 nm or less was collected using a bag filter and separated from the glass particles.

**[Table 1]**

| | | | | | (Mass%) | |
|---|---|---|---|---|---|---|
| Component | SiO₂ | Al₂O₃ | CaO | MgO | TiO₂ | Others |
| Content | 62.12 | 10.98 | 21.44 | 2.85 | 1.50 | Remainder |

### (Example 2)

Glass particles were obtained in the same manner as in Example 1, except that one of the pulverization conditions was changed, specifically, the size of balls put in the ball mill was larger.

### (Comparative Example 1)

Glass particles were obtained in the same manner as in Example 2, except that the spheroidization conditions were changed. The burner combustion temperature of the spheroidizing system was adjusted within the range of 2400 to 2700°C.

### (Comparative Example 2)

Glass particles were obtained in the same manner as in Example 1, except that glass particles of a commercially-available soda-lime glass were used and one of the pulverization conditions was changed, specifically, the ball size was even larger than that in Example 2. Note that the soda-lime glass used has a softening point of approximately 730°C.

### (Comparative Examples 3, 4)

The measurements were performed using silica (L-51 by AGC Si-Tech Co., Ltd.) having a spherical shape in Comparative Example 3 and silicone (KSP-100 by Shin-Etsu Chemical Co., Ltd.) having a spherical shape in Comparative Example 4 instead of the glass particles.

The measurement results are summarized in Table 2.

**[Table 2]**

| | Material | D10 | D50 | D90 | D90/D10 | D50/2 (%) | Feel | Dryness | Shape maintenance | Hardness A | Hardness F |
|---|---|---|---|---|---|---|---|---|---|---|---|
| Ex. 1 | Calcium aluminum borosilicate | 1.3 | 4.8 | 9.1 | 6.77 | 20.5 | Soft | G | G | 4.8 | 97.7 |
| Ex. 2 | Calcium aluminum borosilicate | 1.8 | 10.1 | 19.1 | 10.61 | 16.9 | Soft | G | G | 2.3 | 95.5 |
| Comp. Ex. 1 | Calcium aluminum borosilicate | 4.9 | 10.2 | 18.8 | 3.84 | 7.2 | Smooth | F | NG | 1.1 | 55.9 |
| Comp. Ex. 2 | Soda-lime glass | 12.2 | 19.0 | 31.7 | 2.60 | 3.3 | Smooth | F | NG | 0.8 | 50.8 |
| Comp. Ex. 3 | Silica | 4.1 | 5.9 | 8.7 | 2.12 | 0.31 | Smooth | NG | NG | - | - |
| Comp. Ex. 4 | Silicone | 3.6 | 5.0 | 9.3 | 2.59 | 1.97 | Soft | G | NG | - | - |

| | | | | | | | | | | | |
|---|---|---|---|---|---|---|---|---|---|---|---|
| •The units of D10, D50, and D90 are µm. | | | | | | | | | | | |

For Comparative Examples 3 and 4, no measurement values of the hardnesses were obtained because the press-molded bodies broke in the hardness measurements. According to SEM observation, the glass powders of Examples 1 and 2 each consisted substantially of a solid particle having a perfect spherical shape. The glass powders of Comparative Examples 1 and 2 each had a perfect spherical shape, either. FIG. 1 shows a SEM image of the glass powder of Example 1.

Subsequently, foundations were produced using the powders of Example 1 and Comparative Examples 1 and 3 and were evaluated. The evaluation methods were as described above. Table 3 shows the formulations of the foundations and the evaluation results.

**[Table 3]**

| (Part by mass) | | | | | |
|---|---|---|---|---|---|
| Type | Product name (raw material name) | Manufacturer | Formulation 1 | Formulation 2 | Formulation 3 |
| Extender pigment | JA43-R (talc) | ASADA MILLING CO., LTD. | 35.34 | 35.34 | 35.34 |
| | Sericite FSE (sericite) | Sanshin Mining Ind. Co., Ltd. | 15.00 | 15.00 | 15.00 |
| | NK-10G (synthetic mica) | NIHON KOKEN KOGYO CO., LTD. | 12.00 | 12.00 | 12.00 |
| White pigment | MP-1133 (titanium oxide) | Tayca Corporation | 8.00 | 8.00 | 8.00 |
| Colored pigment | C33-9001 (yellow iron oxide) | DIC | 3.10 | 3.10 | 3.10 |
| | C33-8001 (red iron oxide) | DIC | 0.86 | 0.86 | 0.86 |
| | C33-7001 (black iron oxide) | DIC | 0.28 | 0.28 | 0.28 |
| Powder having spherical shape | Example 1 | | 15.00 | | |
| | Comparative Example 1 | | | 15.00 | |
| | Comparative Example 3 | | | | 15.00 |
| Oil | Silicon KF-56 (methylphenyl polysiloxane) | Shin-Etsu Silicones | 1.00 | 1.00 | 1.00 |
| | Silicon KF-96A-100cs (methylpolysiloxane) | Shin-Etsu Silicones | 1.00 | 1.00 | 1.00 |
| | COSMOL 222 (isostearyl alcohol) | The Nisshin OilliO Group, Ltd. | 2.00 | 2.00 | 2.00 |
| | T.I.O (triethylhexanoin) | NIPPON FINE CHEMICAL CO., LTD. | 2.00 | 2.00 | 2.00 |
| Surfactant | COSMOL 182V (sorbitan sesquiisostearate) | The Nisshin OilliO Group, Ltd. | 1.00 | 1.00 | 1.00 |
| UV-absorber | Nomucoat TAB (octyl methoxycinnamate, tocopherol) | The Nisshin OilliO Group, Ltd. | 3.00 | 3.00 | 3.00 |
| Antioxidant | E-Mix D (natural vitamin E) | Eisai Food & Chemical Co., Ltd. | 0.02 | 0.02 | 0.02 |
| Antiseptic agent | Ethylparaben | UENO FINE CHEMICALS INDUSTRY, LTD. | 0.40 | 0.40 | 0.40 |
| Total | | | 100.00 | 100.00 | 100.00 |
| Evaluation | | Soft/Smooth | Soft | Smooth | Smooth |
| | | Dryness | G | G | NG |
| | | Hardness | 8.2 | 2.1 | 1.1 |

## Claims

1. A glass powder, wherein D50 is 1 µm or more and 15 µm or less and D50/2 is 8% or more, where D50 is a particle diameter at which a cumulative volume from a small-particle side is 50% in a particle size distribution measured by a laser diffraction-scattering method, and D50/2 is a proportion of a cumulative volume from the small-particle side up to a particle size equal to 1/2 of the D50 in the particle size distribution.

2. The glass powder according to claim 1, having a hardness A of 1.5 or more as measured for the glass powder molded into a cylindrical press-molded body having a height of 5 mm, the press-molded body being obtained by filling a mold having a cavity in a shape of a cylinder with the glass powder and applying a 4 MPa pressure to the filled glass powder in a height direction of the cylinder, the hardness A being measured using a type A durometer specified in Japanese Industrial Standards (JIS) K 6253-2012.

3. A glass powder having a hardness A of 1.5 or more as measured for the glass powder molded into a cylindrical press-molded body having a height of 5 mm, the press-molded body being obtained by filling a mold having a cavity in a shape of a cylinder with the glass powder and applying a 4 MPa pressure to the filled glass powder in a height direction of the cylinder, the hardness A being measured using a type A durometer specified in Japanese Industrial Standards (JIS) K 6253-2012.

4. The glass powder according to claim 1 or 3, consisting substantially of a glass particle having a spherical shape, where the spherical shape is a shape having a ratio R2/R1 of 0.8 or more, the ratio R2/R1 being a ratio of a diameter R2 orthogonal to a longest diameter R1 to the diameter R1 in an observation image of the glass particle observed using a scanning electron microscope.

5. The glass powder according to claim 1 or 3, wherein a ratio D90/D10 of D90 to D10 is 4.0 or more, where D10 is a particle diameter at which a cumulative volume from a small-particle side is 10% in a particle size distribution measured by a laser diffraction-scattering method, and D90 is a particle diameter at which the cumulative volume from the small-particle side is 90% in the particle size distribution.

6. The glass powder according to claim 1 or 3, having a glass composition having a softening point of 780°C or higher.

7. The glass powder according to claim 1 or 3, having a hardness F of 60 or more as measured for the glass powder molded into a cylindrical press-molded body having a height of 5 mm, the press-molded body being obtained by filling a mold having a cavity in a shape of a cylinder with the glass powder and applying a 4 MPa pressure to the filled glass powder in a height direction of the cylinder, the hardness F being measured using an ASKER durometer type F.

8. A cosmetic comprising the glass powder according to claim 1 or 3.
